# EUROPEAN PATENT APPLICATION

(11) **EP 4 112 643 A1**
(43) Date of publication of application: **04.01.2023**
(21) Application number: 21761657.2
(22) Date of filing: 25.02.2021
(51) Int. Cl.: C07K 16/28, C12N 15/09, A01K 67/027, A61P 35/00, A61K 39/00

(54) **MONOCLONAL ANTIBODY SPECIFICALLY BINDING TO TIM-3 AND USES THEREOF**

(30) Priority: 25.02.2020 KR 20200023273
(71) Applicant: National Cancer Center, Goyang-si, Gyeonggi-do 10408 (KR)
(72) Inventor: PARK, Eunjung, Seoul 06601 (KR)
(74) Representative: J A Kemp LLP
(86) International application number: PCT/KR2021/002369
(87) International publication number: WO 2021/172890

(57) **Abstract**

The present invention relates to a monoclonal antibody or antigen-binding fragment thereof that specifically binds to TIM-3 and uses thereof.

The monoclonal antibody, hTIM-3_NCC1, of the present invention specifically recognizes human and mouse cells expressing TIM-3, and it can be useful for various fields such as diagnosis of diseases mediated by TIM-3 expressing cells as well as prevention or treatment thereof.

## Description

### [Technical field]

The present invention relates to a monoclonal antibody or antigen-binding fragment thereof that specifically binds to TIM-3 and uses thereof.

### [Background Art]

T cell immunoglobulin and mucin domain-3 (TIM-3) is a transmembrane glycoprotein composed of an immunoglobulin variable domain, a mucin domain, and a cytoplasmic tail with a tyrosine phosphorylation motif. Initially, TIM-3 was proposed as a T helper type 1 (Th1)-specific protein selectively expressed in terminally differentiated Th1 cells, but as a result of further studies, TIM-3 has been shown to be expressed on Th17 cells, regulatory T (Treg) cells, and even non-T cells such as dendritic cells (DC), natural killer (NK) cells, monocytes and macrophages. This suggests that TIM-3 has various immune functions depending on specific cell types and cell states. In particular, TIM-3 seems to play both positive and negative roles depending on the situation. For example, TIM-3 can attenuate the response of CD4+ and CD8+ T cells and inhibit T cell activation to induce peripheral tolerance. However, in certain cases TIM-3 may contribute to the elimination of pathological stimuli by participating in the activation of various innate immune cells, including quiescent macrophages. A recent study showed that TIM-3 promoted short term effector T cells during viral infection. It has also been reported that dysregulation of TIM-3 expression responds excessively or inappropriately in immune cells.

Experimental and clinical studies have implicated TIM-3 in many diseases, including autoimmune diseases, chronic infections, and ischemia. Alterations and dysregulation of TIM-3 expression have been associated with the onset and severity of pathological conditions in patients with multiple sclerosis (MS) and patients infected with human immunodeficiency virus (HIV) or hepatitis virus, and particularly they have been associated with high levels of expression of IFN-y and TNF-α in MS patients as well as distortion of Th1-induced Th2 responses in allergic diseases. Moreover, blockade of TIM-3 has been shown to affect the pathological severity of experimental allergic encephalomyelitis (EAE) and the pathogenesis of diabetes in non-obese diabetic (NOD) mice. It has been shown that blockade of TIM-3 signaling restored proliferation and increased cytokine production in HIV-specific T cells. In these TIM-3-related diseases, negative modulation of Th1 or Th17 immunity by TIM-3 is likely to lead to pathological conditions such as, T cell dysfunction or depletion of T cells, distortion of Th2 responses to Th1 signaling, and proinflammatory states of Th1 responses. However, little is known about the detailed characterization and role of TIM-3 in certain immune states and distinct diseases, particularly in innate immune-related pathologies.

TIM-3 is also receiving attention as a possible target for immune modulation of cancer. In recent years, much research has been done on immune checkpoint molecules and immune surveillance based on cancer growth and eradication, which has led to the development of numerous therapeutic strategies, including therapeutic antibodies targeting immune checkpoint molecules. Studies have suggested that TIM-3 expression is associated with several cancers and that TIM-3 plays a role in regulating tumor growth. For example, TIM-3 is highly expressed on CD4+ and CD8+ tumor-infiltrating T cells from patients with non-small cell lung cancer, and TIM-3 expression on CD4+ T cells is associated with poor clinicopathological parameters such as nodular metastasis and advanced cancer. It was found to be characteristically expressed in tumor cells in patients with renal and hepatocellular carcinoma.

Korean Patent No. 10-2146319 relates to a bispecific antibody specific for PD-1 and TIM-3, and it provides a bispecific antibody that binds to TIM3 with a lower binding affinity compared to binding to PD1, comprising a first antigen-binding site specifically binding to PD1 and a second antigen binding site specifically to TIM3.

However, there is still a need for research on antibodies that specifically bind to TIM-3 with high affinity and can be used for monitoring TIM-3 itself or for diagnosing diseases related to TIM-3 expression.

### [Disclosure]

### [Technical Problem]

Accordingly, the present inventors have made and selected the monoclonal antibody of the present invention as a result of earnest efforts to provide an antibody that specifically binds to TIM-3 with high affinity, and completed the present invention.

Accordingly, it is an object of the present invention to provide a monoclonal antibody or antigen-binding fragment thereof that specifically binds to TIM-3, and uses thereof.

### [Technical solution]

The present invention provides a monoclonal antibody or antigen-binding fragment thereof that specifically binds to TIM-3 comprising: a heavy chain variable region including a heavy chain CDR1 represented by an amino acid sequence of SEQ ID NO: 1, a heavy chain CDR2 represented by an amino acid sequence of SEQ ID NO: 2, and a heavy chain CDR3 represented by an amino acid sequence of SEQ ID NO: 3; and a light chain variable region including a light chain CDR1 represented by an amino acid sequence of SEQ ID NO: 4, a light chain CDR2 represented by an amino acid sequence of SEQ ID NO: 5, and a light chain CDR3 represented by an amino acid sequence of SEQ ID NO: 6.

According to a preferred embodiment of the present invention, the antibody may comprise a heavy chain variable region represented by an amino acid sequence of SEQ ID NO: 7; and a light chain variable region represented by an amino acid sequence of SEQ ID NO: 8.

According to a preferred embodiment of the present invention, the heavy chain variable region and the light chain variable region may be linked by a linker represented by an amino acid sequence of SEQ ID NO: 9.

According to a preferred embodiment of the present invention, the antibody may be a humanized antibody or a human antibody.

In addition, the present invention also provides a nucleic acid molecule encoding the heavy chain variable region of the monoclonal antibody or antigen-binding fragment thereof.

According to a preferred embodiment of the present invention, the nucleic acid molecule may comprise the nucleotide sequence of SEQ ID NO: 16 or the nucleotide sequence of SEQ ID NO: 17.

In addition, the present invention provides a recombinant vector comprising the nucleic acid molecule encoding the heavy chain variable region, the nucleic acid molecule encoding the light chain variable region, or all of the nucleic acid molecules.

In addition, the present invention provides a host cell comprising the recombinant vector.

In addition, the present invention provides a method for producing a monoclonal antibody or antigen-binding fragment thereof that specifically binds to TIM-3, comprising culturing the host cell.

In addition, the present invention provides a kit for measuring a TIM-3 expression level comprising the monoclonal antibody or antigen-binding fragment thereof.

In addition, the present invention provides a method for monitoring a TIM-3 expression level comprising:
i) contacting the monoclonal antibody or antigen-binding fragment thereof of claim 1 with a biological sample isolated from a subject; and
ii) measuring a TIM-3 expression level bound to the monoclonal antibody or antigen-binding fragment thereof in the biological sample through antigen-antibody complex formation.

According to a preferred embodiment of the present invention, the biological sample may be at least one selected from the group consisting of whole blood, serum, plasma, saliva, urine, tissue and cell.

### [Advantageous Effects]

The monoclonal antibody, such as, hTIM-3_NCC1, of the present invention specifically recognizes human and mouse cells expressing TIM-3, and it can be useful for various fields such as diagnosis of diseases mediated by TIM-3 expressing cells as well as prevention or treatment thereof.

### [Description of Drawings]

Fig. 1 shows ELISA results of antibody candidates specific for TIM-3. They correspond to scFv-TIM3#1 to scFv-TIM3#12 in order from left.
Fig. 2 shows the amino acid sequence and CDR region sequence of the selected hTIM-3_NCC1 antibody.
Fig. 3 shows that the selected hTIM-3_NCC1 antibody specifically binds to human TIM-3.
Fig. 4 shows that the selected hTIM-3_NCC1 antibody specifically binds to human TIM-3 expressed by Jurkat cells.
Fig. 5 shows that the selected hTIM-3_NCC1 antibody specifically binds to human TIM-3.
Fig. 6 shows that the selected hTIM-3_NCC1 antibody responds and specifically binds to human TIM-3 in human PBMCs better than a commercially available anti-human TIM-3 antibody (BV421 α-Human TIM-3, BD#565553).
Fig. 7 shows that the selected hTIM-3_NCC1 antibody can also bind to mouse TIM-3. hIgG Fc refers to APC- hIgG Fc, and α-hTIM-3 refers to hTIM-3_NCC1 of the present antibody.
Fig. 8 shows that the selected hTIM-3_NCC1 antibody can also bind to mouse TIM-3. In the lower graph, Rat Isotype-PE (eBioscience, cat#12-4321) refers to an isotype antibody of α-mTIM-3-PE antibody (eBioscience, cat#12-5870), and APC- hIgG Fc (Biolegend, cat#409305) refers to a secondary antibody control against α-hTIM-3-scFv-hIgG (hTIM-3_NCC1).

### [Mode of the Invention]

Hereinafter, the present invention will be described in more details.

In the present invention, the "antigen" may mean a molecule or part of a molecule capable of binding by an antibody, and it can be used in an animal to produce an antibody capable of binding to an epitope of an antigen. An antigen may have more than one epitope.

In the present invention, the "antibody" or "Ab" may mean an immunoglobulin molecule capable of recognizing and binding to a specific target or antigen, such as a carbohydrate, polynucleotide, lipid, polypeptide, etc., via one or more antigen recognition sites, located in the variable region of an immunoglobulin molecule. "Antibody" may mean a monoclonal antibody; polyclonal antibody; "antigen binding fragments" of antibodies that retain the ability to specifically bind to a particular antigen (e.g., TIM-3), such as Fab, Fab', F(ab')2, Fd, Fv, Fc etc; isolated complementarity determining region (CDR); bispecific antibody; heteroconjugate antibody or mutants thereof; fusion protein having an antibody, or antigen-binding fragment thereof (e.g., domain antibody); single chain (ScFv) and single domain antibody (e.g., shark and camelid antibody); maxibody, minibody, intrabody, diabody, triabody, tetrabody, v-NAR and bis-scFv; humanized antibody; chimeric antibody; or any kind of antibody, such as, antibody having modified configuration of immunoglobulin molecule comprising antigen recognition site of the required specificity (e.g., glycosylation variant of antibody, amino acid sequence variant of antibody, and covalently modified antibody), but not limited thereto. The antibody may be of murine, rat, human, or of any other origin (including chimeric or humanized antibodies).

The "CDR (complementarity determining region)" may mean an amino acid sequence of a hypervariable region of immunoglobulin heavy and light chains. The heavy chain and light chain each contain three CDRs (CDRH1, CDRH2 and CDRH3; CDRL1, CDRL2 and CDRL3), which provide key contact residues for the antibody to bind antigen or epitope.

An antibody or polypeptide that "specifically binds" to a particular target or antigen (e.g., TIM-3) is a term well understood in the art, and methods of determining such specific binding are also widely known in the art. A particular molecule is said to exhibit "specific binding" when it reacts or associates with a particular cell or substance more frequently, more rapidly, with greater persistence and/or with greater affinity than it does with another cell or substance. A particular antibody "specifically binds" to a particular target or antigen if it binds with greater affinity, binding activity, more rapidly and/or with greater persistence than it binds other substances.

In the present invention, the "vector" includes a nucleic acid molecule capable of carrying another nucleic acid to which it has been linked. One form of vector is a "plasmid," which refers to a circular double-stranded DNA loop to which additional DNA segments can be bound. Another form of vector is a viral vector, in which additional DNA segments can be bound to the viral genome. Certain vectors are capable of autonomous replication in the host cell into which they are introduced (e.g., bacterial vector having replication of bacterial origin and episomal mammalian vector). Other vectors (e.g., non-episomal mammalian vector) may be integrated into the genome of a host cell upon introduction into the host cell, thereby being replicated along the host genome. In addition, some vectors are capable of directing the expression of genes to which they are operatively linked. Such vector is referred to herein as "recombinant expression vector (or simply, "expression vector"). In general, expression vectors useful in recombinant DNA technology often exist in the form of plasmids. As used herein, "plasmid" and "vector" can be used interchangeably because plasmid is the most commonly used form of vector. However, the present invention can include other forms of expression vectors with equivalent functions, such as viral vectors (e.g., replication-deficient retrovirus, adenovirus and adeno-associated virus).

In the present invention, the "host cell" may be used to mean a cell capable of expressing a selected gene of interest after being transformed or transformed with a nucleic acid sequence. The host cell may include the progeny of the parent cell whether the progeny is identical in morphology or genetic organization to the original parent, so long as the selected gene is present.

As described above, the present inventors have completed the present invention as a result of earnest efforts to provide an antibody that can be usefully used for TIM-3 monitoring and disease diagnosis through specific and selective strong binding to TIM-3.

Since the antibody of the present invention, such as hTIM-3_NCC1, can specifically and selectively bind not only human TIM-3 but also mouse TIM-3, it is very suitable for monitoring TIM-3 expression level. Therefore, when using the antibody hTIM-3_NCC1 of the present invention, since the expression level of TIM-3 can be measured very accurately, it can be widely used in the field for checking TIM-3 expression level, for example, the field of diagnosis or predicting the prognosis diseases such as cancer, multiple sclerosis or brain disease or the field of preclinical research using mice.

Accordingly, the present invention provides a monoclonal antibody or antigen-binding fragment thereof that specifically binds to TIM-3 comprising:
a heavy chain variable region including a heavy chain CDR1 represented by an amino acid sequence of SEQ ID NO: 1, a heavy chain CDR2 represented by an amino acid sequence of SEQ ID NO: 2, and a heavy chain CDR3 represented by an amino acid sequence of SEQ ID NO: 3; and
a light chain variable region including a light chain CDR1 represented by an amino acid sequence of SEQ ID NO: 4, a light chain CDR2 represented by an amino acid sequence of SEQ ID NO: 5, and a light chain CDR3 represented by an amino acid sequence of SEQ ID NO: 6.

The "antigen-binding fragment thereof" refers to a fragment having an antigen-binding function, and includes Fab, F(ab'), F(ab')2 and Fv or single-chain antibody molecule, etc. Among the antibody-binding fragments, Fab has one antigen-binding site in a structure having a light chain and heavy chain variable regions, and a light chain constant region and a heavy chain first constant region (CH1). F(ab') differs from Fab in that it has a hinge region comprising one or more cysteine residues at the C-terminus of the heavy chain CH1 domain. F(ab')2 is generated when a cysteine residue in the hinge region of Fab' forms a disulfide bond. Fv refers to the smallest antibody fragment having only a heavy chain variable region and a light chain variable region. Such an antibody fragment can be obtained by using a proteolytic enzyme, for example, by restriction digestion of the entire antibody with papain to obtain Fab, and by digestion with pepsin to obtain a F (ab')2 fragment, preferably can be produced through genetic recombination technology.

According to a preferred embodiment of the present invention, the antibody may comprise a heavy chain variable region represented by an amino acid sequence of SEQ ID NO: 7; and a light chain variable region represented by an amino acid sequence of SEQ ID NO: 8.

According to a preferred embodiment of the present invention, the heavy chain variable region and the light chain variable region may be linked by a linker represented by an amino acid sequence of SEQ ID NO: 9.

According to a preferred embodiment of the present invention, the antibody may be a humanized antibody or a human antibody.

The humanized antibody is engineered to contain an immunoglobulin domain that is even more human-like, which contains only the complementarity determining regions of an animal-derived antibody. This is achieved by carefully examining the sequence of the hypervariable loops of the variable regions of the monoclonal antibody and adapting the sequence to the structure of the human antibody chain.

A fully humanized antibody is an antibody molecule in which the entire sequence of both the light and heavy chains, including the CDRs, arises from human genes.

In addition, the present invention also provides a nucleic acid molecule encoding the heavy chain variable region of the monoclonal antibody or antigen-binding fragment thereof.

The "nucleic acid molecule" has a meaning comprehensively including DNA (gDNA and cDNA) and RNA molecules. Nucleotide, which is a basic structural unit in a nucleic acid molecule, include not only natural nucleotide, but also analogs in which sugar or base sites are modified. The sequence of the nucleic acid molecule encoding the variable region of heavy and light chains of the present invention may be modified. Such modifications include additions, deletions, or non-conservative or conservative substitutions of nucleotides. The nucleic acid molecule of the present invention is to be construed to include a nucleotide sequence exhibiting substantial identity to the above-described nucleotide sequence. The substantial identity is at least 80 % homology, in one specific example at least 90% homology, in another specific example at least 95 % homology when the nucleotide sequence of the present invention and any other sequences are aligned to the maximum correspondence and the aligned sequence is analyzed using an algorithm commonly used in the art.

According to a preferred embodiment of the present invention, the nucleic acid molecule encoding the variable region of heavy chain may comprise the nucleotide sequence of SEQ ID NO: 16, and the nucleic acid molecule encoding the variable region of light chain may comprise the nucleotide sequence of SEQ ID NO: 17.

In addition, the present invention provides a recombinant vector comprising the nucleic acid molecule encoding the heavy chain variable region, the nucleic acid molecule encoding the light chain variable region, or all of the nucleic acid molecules.

The recombinant vector system of the present invention can be constructed through various methods known in the art. The vectors of the present invention can typically be constructed as vectors for cloning or as vectors for expression. In addition, the vector of the present invention can be constructed using a prokaryotic cell or a eukaryotic cell as a host.

The present invention can also provide a host cell comprising the recombinant vector.

The host cell is a cell transformed with the recombinant vector of the present invention. A host cell capable of stably and continuously cloning and expressing the vector of the present invention is known in the art and any host cell may be used, for example, *Escherichia coli, Bacillus* sp. strains such as *Bacillus subtilis* and *B. thuringensis, Streptomyces, Pseudomonas* (e.g., *Pseudomonas putida*), *Proteus Mirabilis* or *Staphylococcus* (e.g., *Staphylococcus carnosus*).

The present invention may also provide a method for producing a monoclonal antibody or antigen-binding fragment thereof that specifically binds to TIM-3, comprising culturing the host cell.

The culture of host cells for production of the antibody or antigen-binding fragment thereof may be performed according to a suitable medium and culture conditions known in the art. Such a culture process can be easily adjusted and used by those skilled in the art according to the selected strain. Cell culture can be divided into suspension culture and adherent culture according to the cell growth method, and batch, fed-batch and continuous culture methods according to the culture method. The medium used for culture should suitably satisfy the requirements of a particular strain.

The present invention may also provide a kit for measuring TIM-3 expression level comprising the monoclonal antibody or antigen-binding fragment thereof.

The expression level can be measured by a method capable of measuring antigen-antibody binding, and the method may include any immunological analysis method. Such methods are known in the art and include, for example, Western blot, ELISA, radioimmunoassay, radioimmunoassay. diffusion method, immunofluorescence assay, immunoblot, Oucreroni immunodiffusion method, rocket immunoelectrophoresis, tissue immunostaining, immunoprecipitation assay, complement fixation assay, immunochromatographic assay, FACS, or protein chip.

Tools or reagents used in immunological assays may include suitable carriers or supports, labels capable of generating a detectable signal, solubilizing agents, detergents, and stabilizers, and the like. Suitable carriers include, but are not limited to, a substrate capable of measuring enzyme activity when the labeling substance is an enzyme, a suitable buffer solution, a secondary antibody labeled with a chromogenic enzyme or a fluorescent substance, a chromogenic substrate, and a reaction stop agent, etc.

The label includes any composition detectable by spectroscopic, photochemical, biochemical, immunochemical, electrical, optical or chemical means. Suitable labels include, but are not limited to, magnetic beads, fluorescent dyes (e.g., fluorescein isothiocyanate, Texas red, rhodamine, green fluorescent protein, red fluorescent protein, yellow fluorescent protein, etc.), radioactive labels (e.g., ³H, ¹²⁵I, ³⁵S, ¹⁴C or ³²P), enzymes (e.g., mustard radish peroxidase, alkaline phosphatase, luciferase and any enzyme which can be commonly used for enzyme-linked immunosorbent assay (ELISA)) and colorimetric labels such as colloidal gold or colored glass or plastic bead ( e.g., polystyrene, polypropylene, latex, etc.).

The antibody included in the kit of the present invention may be immobilized on a suitable carrier or support using various methods as disclosed in the literature, and examples of suitable carriers or supports include PBS, polystyrene, polyethylene, polypropylene, polyester, polyacrylonitrile, fluororesin, agarose, cellulose, nitrocellulose, dextran, sephadex, sepharose, liposome, carboxymethyl cellulose, polyacrylamide, polysterin, gabbro, filter paper, ion exchange resin, plastic film, plastic tube, polyamine-methyl vinyl-ether-maleic acid copolymer, amino acid copolymer, ethylene-maleic acid copolymer, nylon, metal, glass, glass beads, or magnetic particles, and the like. Other solid substrates include cell culture plates, microplates, microarrays, ELISA plates, tubes, and polymeric membranes. The support may have any possible shape, for example spherical (bead), cylindrical (test tube or well inner surface), planar (sheet, test strip).

In addition, the present invention provides a kit for measuring a TIM-3 expression level comprising the monoclonal antibody or antigen-binding fragment thereof.

In addition, the present invention provides a method for monitoring a TIM-3 expression level comprising:
i) contacting the monoclonal antibody or antigen-binding fragment thereof of claim 1 with a biological sample isolated from a subject; and
ii) measuring a TIM-3 expression level bound to the monoclonal antibody or antigen-binding fragment thereof in the biological sample through antigen-antibody complex formation.

The subject of step i) may be a mammal including a human.

According to a preferred embodiment of the present invention, the biological sample may be any one or more selected from the group consisting of whole blood, serum, plasma, saliva, urine, tissue and cells.

Hereinafter, the present invention will be described in more detail through examples. These examples are only for illustrating the present invention, and it is obvious to those of ordinary skill in the art that the scope of the present invention is not to be interpreted as being limited by these examples.

### <Example 1> Selection and sequencing of antibodies that specifically bind to TIM-3

TIM-3 specific antibodies (hTIM-3_NCC1, scFv-TIM3 #4) were selected through screening and ELISA reaction of human scFv (naive) library using human TIM-3 antigen.

Specifically, the selection of antibodies specific for human TIM-3 antigen, Tim-3-His tag protein, was performed using a biopanning method known in the art. First, in order to coat the TIM-3 antigen, TIM-3-His tag (ACRObiosystems Inc.) was diluted in PBS at 2 µg /ml and dispensed on a plate by 100 µl per well. After the antigen was shaken off, 100 µl of 5% skim milk in PBS solution was added per well and reacted at room temperature for 4 hours, followed by washing with PBS 4 times or more. ScFv binding to TIM-3-His tag by bio-panning antibody library of scFv expressed on the surface of M13 phage (M13 phage antibody library) in 96-well plate to which Tim-3 protein antigen is attached was collected.

12 TIM-3 specific antibody candidates (ScFV-TIM3#1 to ScFV-TIM3#12) obtained through the human ScFv (naive) library screening were diluted in 1% skim milk in TBST, respectively, and treated in each well. After reacting at room temperature for 1 hour, it was washed 3 times with IX PBST. Secondary antibody (anti-human IgG HRP, 1:5000) was treated and reacted by shaking at room temperature for 1 hour, washed 6 times with 1X PBST, and then treated with 50 ml of TMB solution for color development at room temperature for 30 minutes. Finally, 100 ml of IN H₂SO₄ stop solution was treated for 10 minutes to terminate the reaction, and then absorbance was measured at 450 nm.

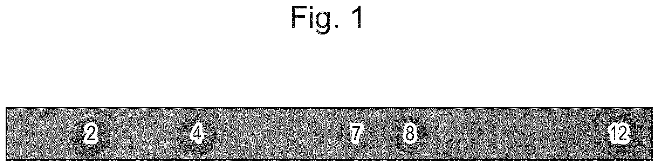

As a result, as shown in Fig. 1, ScFV-TIM3#2, ScFV-TIM3#4, ScFV-TIM3#7, ScFV-TIM3#8 and ScFV-TIM3#12 antibodies were identified among 12 candidates, and ScFV-TIM3#4 (a-hTIM-3-scFv-hIgG) showing the strongest signal was selected.

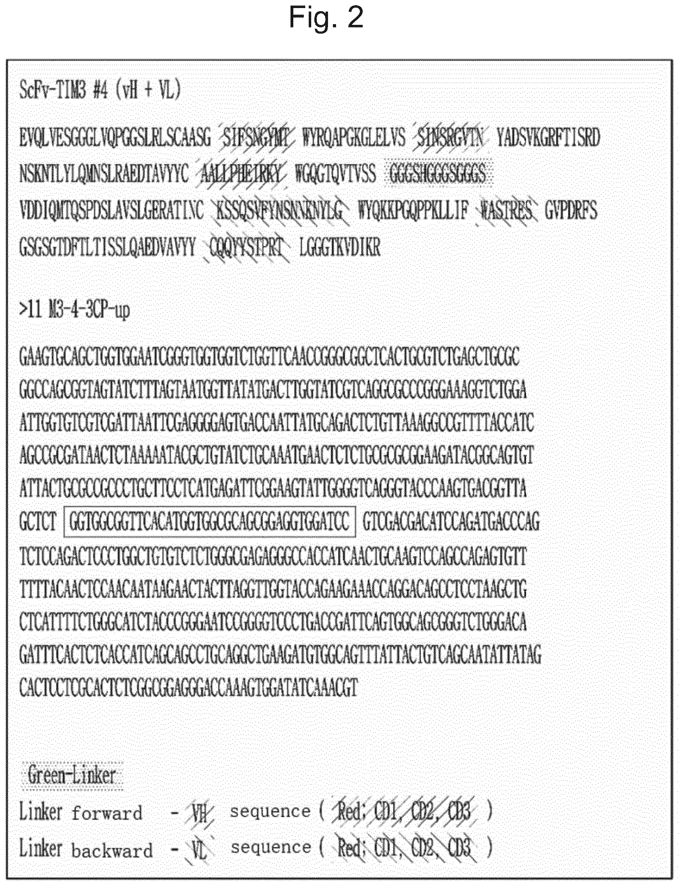

The selected ScFV-TIM3#4 clone was named hTIM-3_NCC1, and its base and amino acid sequences were analyzed. According to the results of sequencing, the sequence information for antibody CDR regions, i.e. the heavy chain variable region and light chain variable region was confirmed (Table 1, Fig. 2).

**[Table 1]**

| Area | Sequence | SEQ ID NO: |
|---|---|---|
| heavy chain CDR1 | SIFSNGYMT | 1 |
| heavy chain CDR2 | SINSRGVTN | 2 |
| heavy chain CDR3 | AALLPHEIRKY | 3 |
| light chain CDR1 | KSSQSVFYNSNNKNYLG | 4 |
| light chain CDR2 | WASTRES | 5 |
| light chain CDR3 | CQQYYSTPRT | 6 |
| heavy chain variable region | EVQLVESGGGLVQPGGSLRLSC AASGSIFSNGYMTWYRQAPGKG LELVSSINSRGVTNYADS VKGRFTISRDNSKNTLYLQMNS | 7 |
| | LRAEDTAVYYCAALLPHEIRKY WGQGTQVTVSS | |
| light chain variable region | VDDIQMTQSPDSLAVSLGERATI NCKSSQSVFYNSNNKNYLGWY QKKPGQPPKLLIFWASTRESGVP DRFSGSGSGTDFTLTISSLQAED VAVYYCQQYYSTPRTLGGGTKV DIKR | 8 |
| linker | GGGSHGGGSGGGS | 9 |
| heavy chain CDR1 | TCACTGCGTCTGAGCTGCGCGG CCAGC | 10 |
| heavy chain CDR2 | GCGCCCGGAAAGGTCTGGAA TTGGTG | 11 |
| heavy chain CDR3 | GCCGCCCTGCTTCCTCATGAGA TTCGGAAGTAT | 12 |
| light chain CDR1 | AAGTCCAGCCAGAGTGTTTTTT ACAACTCCAACAATAAGAACT ACTTAGGT | 13 |
| light chain CDR2 | TGGGCATCTACCCGGGAATCC | 14 |
| light chain CDR3 | TGTCAGCAATATTATAGCACTC CTCGCACT | 15 |
| heavy chain variable region | GAAGTGCAGCTGGTGGAATCG GGTGGTGGTCTGGTTCAACCGG | 16 |
| | GCGGCTCACTGCGTCTGAGCTG CGCGGCCAGCGGTAGTATCTTT AGTAATGGTTATATGACTTGGT ATCGTCAGGCGCCCGGGAAAG GTCTGGAATTGGTGTCGTCGAT TAATTCGAGGGGAGTGACCAA TTATGCAGACTCTGTTAAAGGC CGTTTTACCATCAGCCGCGATA ACTCTAAAAATACGCTGTATCT GCAAATGAACTCTCTGCGCGC G GAAGATACGGCAGTGTATTAC TGCGCCGCCCTGCTTCCTCATG AGATTCGGAAGTATTGGGGTC AGGGTACCCAAGTGACGGTTA GCTCT | |
| light chain variable region | GTCGACGACATCCAGATGACC CAGTCTCCAGACTCCCTGGCTG TGTCTCTGGGCGAGAGGGCCA CCATCAACTGCAAGTCCAGCC AGAGTGTTTTTTACAACTCCAA CAATAAGAACTACTTAGGTTG GTACCAGAAGAAACCAGGACA | 17 |
| | GCCTCCTAAGCTGCTCATTTTC TGGGCATCTACCCGGGAATCC GGGGTCCCTGACCGATTCAGTG GCAGCGGGTCTGGGACAGATT TCACTCTCACCATCAGCAGCCT GCAGGCTGAAGATGTGGCAGT TTATTACTGTCAGCAATATTAT AGCACTCCTCGCACTCTCGGCG GAGGGACCAAAGTGGATATCA AACGT | |
| linker | GGTGGCGGTTCACATGGTGGC GGCAGCGGAGGTGGATCC | 18 |

### <Example 2> Confirmation of specific binding of the selected antibody to human TIM-3

To confirm the specificity of the antibody hTIM-3_NCC1 selected in < Example 1> for TIM-3, ELISA analysis was performed.

Specifically, in order to coat the TIM-3 antigen, TIM-3-His tag (ACRObiosystems Inc.) was diluted in PBS at 2 µg /ml and dispensed to the plate at 100 µl per well. After the antigen was shaken off, 100 µl of 5% skim milk in PBS solution was added per well, reacted at room temperature for 4 hours, and then washed 4 times or more with PBS. hTIM-3_NCC1 was diluted in 1% skim milk in TBST and treated in each well, followed by reaction at room temperature for 1 hour, and then washed 3 times with 1 X PBST. Secondary antibody (anti-human IgG HRP, 1:5000) was treated and reacted by shaking at room temperature for 1 hour, washed 6 times with IX PBST, and then treated with 50 ml of TMB solution for color development at room temperature for 30 minutes. Finally, 100 ml of IN H₂SO₄ stop solution was treated for 10 minutes to terminate the reaction, and then absorbance was measured at 450 nm. The following table 2 shows the ELISA test conditions.

**[Table 2]**

| | |
|---|---|
| Antigen | TIM-3-His-tag, 200 ng/well |
| Primary Ab | Anti-TIM-3-hscFv-hIgG1 |
| Secondary Ab-HRP | 1:3,000 |
| TMB substrate incubation | 10 min |
| Measurement filter | 450nm |
| plate reader | Infinite F50 |

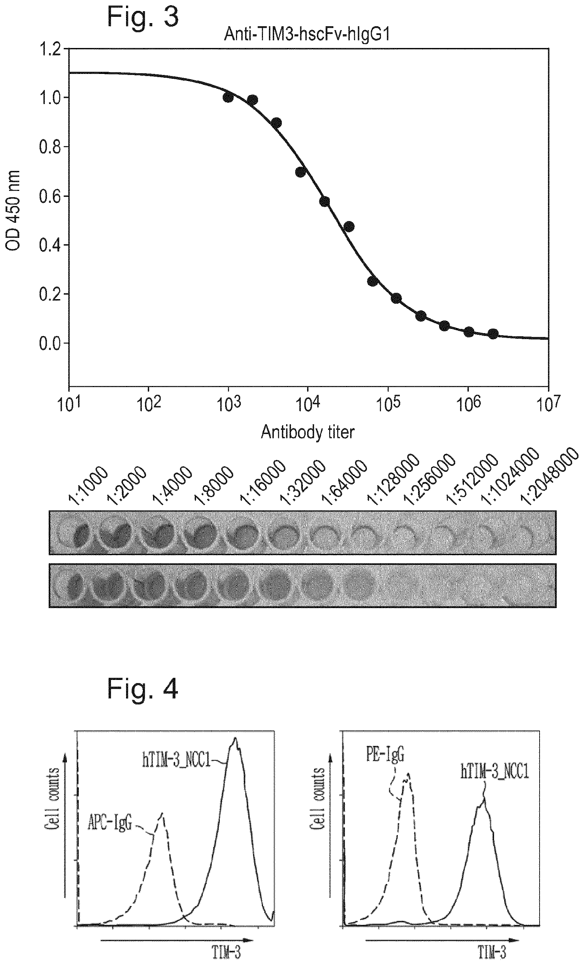

As a result, it was confirmed that hTIM-3_NCC1 specifically bound to TIM-3 as shown in Fig. 3. As a result of ELISA QC, detection was possible up to 1 : 128,000, and at this time, the amount of antigen coated on the immunoplate was about 0.2 µg (200ng/ well) per well.

### <Example 3>

### Confirmation of Selective Binding of Selected Antibodies to Human TIM-3

### <3-1> Confirmation of reactivity of human T-cell lymphoma cells to human TIM-3

FACS analysis was performed to confirm the specificity of the antibody hTIM-3_NCC1 selected in < Example 1> for TIM-3.

Specifically, 3×10⁵ Jurkat human immortalized human T lymphocyte cells (T cells leukemia) expressing TIM-3 alone, or 1 µg of the antibody hTIM-3_NCC1 were reacted with them for 30 minutes, and then the surface was strained with a secondary antibody (APC-Human IgG Fc or PE-Human IgG Fc) for 10 minutes and measured by flow cytometry.

As a result, as shown in Fig. 4, it was confirmed that the antibody hTIM-3_NCC1 specifically bound to cells expressing TIM-3.

### Confirmation of reactivity to mouse NIH3T3 cells overexpressing human TIM-3

To confirm the specificity of the antibody hTIM-3_NCC1 selected in < Example 1> for TIM-3, FACS analysis was performed.

Specifically, human TIM-3 was overexpressed in NIH/3T3 mouse cells using Lenti particles encoding TIM3 cDNA prepared using Lenti-Human TIM-3-GFP plasmid DNA (Origene, cat#RC209440L4) and Lenti-control particles-GFP (Origene, cat#PS 100093 V) as follows: ① A control group was Lenti-control particles-GFP group. 10 MOI of Lenti particles was treated in 5×10⁴ NIH3T3 cells together with polybrene, and only the transduced cells were selectively selected using puromycin (NIH3T3- GFP). ② The experimental group was Lenti -Human TIM-3-GFP plasmid DNA group. After transfection with packing DNA into 293FT cells, the medium containing lenti-Human TIM-3-GFP particles obtained 3 days after transfection was treated with polybrene in NIH/3T3. Only cells overexpressing human TIM-3 were selectively selected using puromycin (NIH3T3-GFP-Human TIM-3).

1×10⁵ cells selected above were reacted with 0.1 µg of hTIM-3_NCC1 antibody for 30 minutes, and then stained with secondary antibodies (PE-Human IgG Fc, APC-Human IgG Fc) for 20 minutes, followed by flow cytometry. Expression of human TIM-3 was confirmed by GFP expression, and in cells expressing GFP, expression differences between the transduced groups with Lenti-contorl particles and Lenti - Human Tim-3 particles, respectivelly, were confirmed.

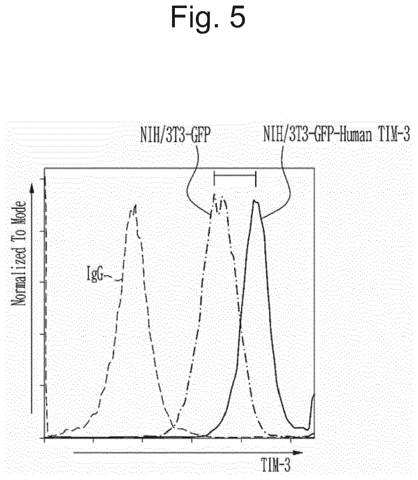

As a result, as shown in [FIG. 5], it was confirmed that the selected antibody hTIM-3_NCC1 reacted specifically to human TIM-3.

### <3-3> Confirmation of reactivity to human PBMC

FACS analysis was performed to compare the reactivity of the antibody hTIM-3_NCC1 (α-hTIM-3-scFv-hIgG) selected in < Example 1> and the currently marketed anti-human TIM-3 antibody to TIM-3.

Specifically, after human blood was collected, only peripheral blood mononuclear cells (PBMCs) were isolated separately by Ficoll-paque density gradient using Ficoll-Paque (GE healthcare, cat#17-5442-02). 1×10⁶ isolated PBMCs, 0.5 µg of the above selected antibody, 1 µg of PE-Cy7-conjugated anti-CD3 antibody (BD, cat#557851, USA) and 0.25 µg of FITC-conjugated anti-CD11b antibody (ebioscience, cat #11-0112, USA) were reacted for 30 minutes, and then the surface was stained with a secondary antibody secondary antibody (PE-Human IgG Fc), and then measured by flow cytometry. A commercially available BV421-conjugated anti-TIM-3 antibody (BV421-Human TIM-3; BD, cat#565553, USA) was used as a positive control. Levels of TIM-3 were measured in CD3^{high}CD11b⁻, CD3^{high}CD11b^{mid}, CD3⁻CD11b^{mid} and CD3⁻CD11b^{high} cells.

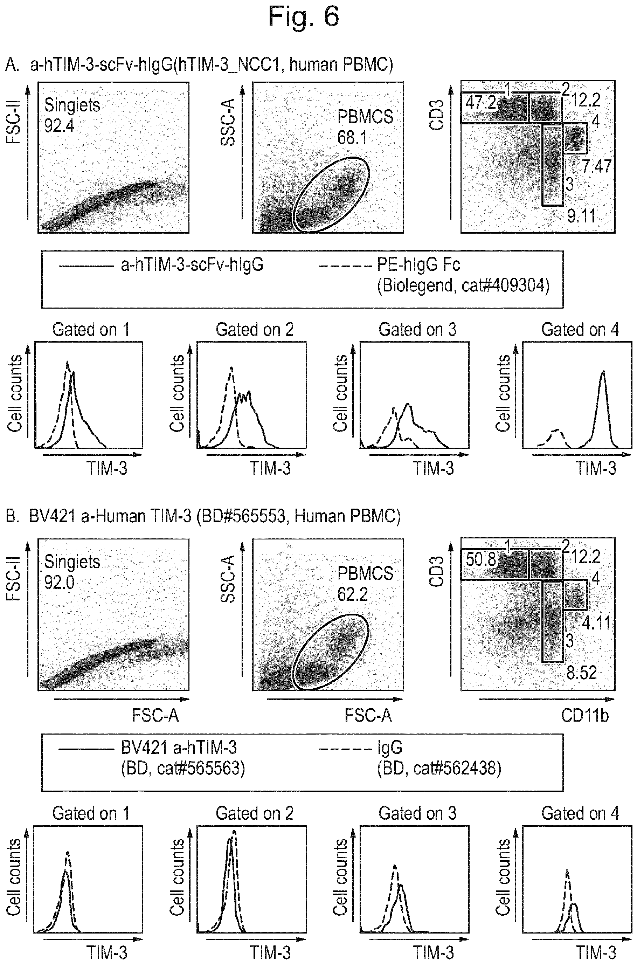

As a result, as shown in Fig. 6, hTIM-3_NCC1 reacted by recognizing TIM-3 expressed by cell groups expressing CD3 and CD11b of human PBMC, and hTIM-3_NCC1 had superior reactivity than a commercially available antibody used as a positive control.

### < Example 4> Confirmation of binding of the selected antibody to mouse TIM-3 and its applicability to preclinical studies

FACS analysis was performed to determine whether the selected antibody, hTIM-3_NCC1, specifically binds to mouse TIM-3 as well as human TIM-3.

Specifically, 0.5 µg of hTIM-3_NCC1 antibody was reacted to mouse primary glial cells for 30 minutes, and then stained with a secondary antibody (APC-human IgG Fc; Biolegend, cat#409305). In addition, mouse CD11b⁺ cells overexpressing mouse TIM-3 (3 × 10⁵ cells) were separated using a-BV421-conjugated anti-CD11b antibody (BD, cat#562605) and then 0.5 µg of hTIM-3_NCC1 antibody and PE-conjugated anti-mouse were reacted for 30 minutes. These cells were surface-stained with a secondary antibody (APC-Human IgG Fc) and then measured by flow cytometry.

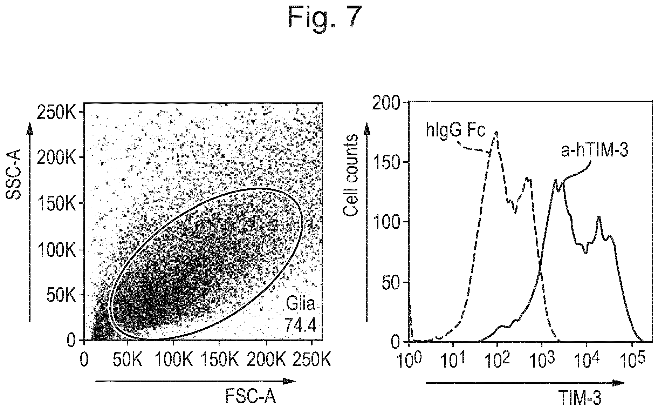

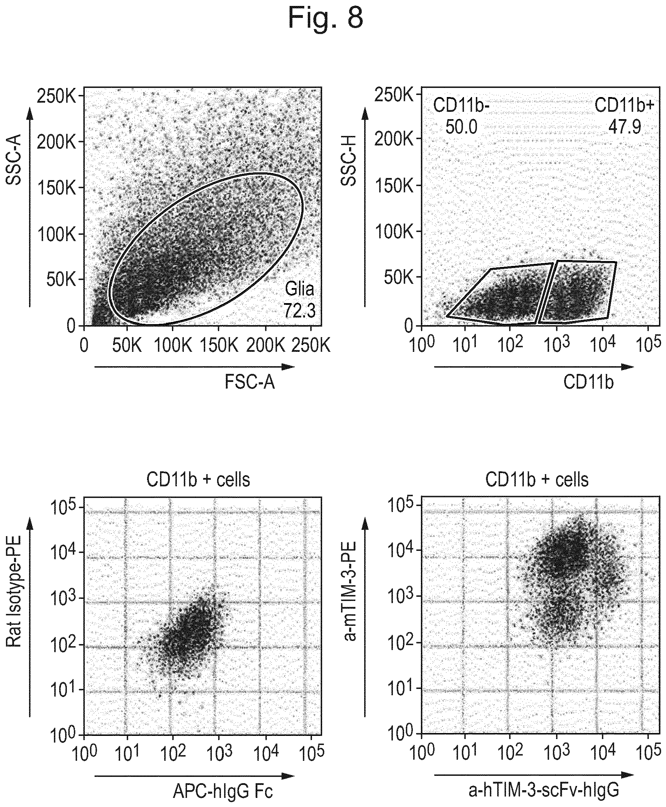

As a result, as shown in Fig. 7, it was confirmed that hTIM-3_NCC1 also responded to TIM-3 expressed in mouse primary glial cells. In addition, as shown in Fig. 8, it was confirmed that the mouse hTIM-3_NCC1 antibody was also reactive with TIM-3 of the mouse cells. These results suggest that the novel antibody of the present invention can be used in preclinical studies such as mice monitoring TIM-3 expression.

## Claims

1. A monoclonal antibody or antigen-binding fragment thereof that specifically binds to TIM-3 comprising:
a heavy chain variable region including a heavy chain CDR1 represented by an amino acid sequence of SEQ ID NO: 1, a heavy chain CDR2 represented by an amino acid sequence of SEQ ID NO: 2, and a heavy chain CDR3 represented by an amino acid sequence of SEQ ID NO: 3; and
a light chain variable region including a light chain CDR1 represented by an amino acid sequence of SEQ ID NO: 4, a light chain CDR2 represented by an amino acid sequence of SEQ ID NO: 5, and a light chain CDR3 represented by an amino acid sequence of SEQ ID NO: 6.

2. The monoclonal antibody or antigen-binding fragment thereof that specifically binds to TIM-3 according to claim 1, wherein the antibody comprises a heavy chain variable region represented by an amino acid sequence of SEQ ID NO: 7; and a light chain variable region represented by an amino acid sequence of SEQ ID NO: 8.

3. The monoclonal antibody or antigen-binding fragment thereof that specifically binds to TIM-3 according to claim 1, wherein the heavy chain variable region and the light chain variable region are linked by a linker represented by an amino acid sequence of SEQ ID NO: 9.

4. The monoclonal antibody or antigen-binding fragment thereof according to claim 1, wherein the antibody is a humanized antibody or a human antibody.

5. A nucleic acid molecule encoding the heavy chain variable region of monoclonal antibody or an antigen-binding fragment thereof of claim 1.

6. The nucleic acid molecule of claim 5, wherein the nucleic acid molecule comprises the nucleotide sequence of SEQ ID NO: 16.

7. A nucleic acid molecule encoding the light chain variable region of monoclonal antibody or an antigen-binding fragment thereof of claim 1.

8. The nucleic acid molecule of claim 7, wherein the nucleic acid molecule comprises the nucleotide sequence of SEQ ID NO: 17.

9. A recombinant vector comprising the nucleic acid molecule encoding the heavy chain variable region of claim 5, the nucleic acid molecule encoding the light chain variable region of claim 7, or all of the nucleic acid molecules.

10. A host cell comprising the recombinant vector of claim 9.

11. A method for producing a monoclonal antibody or antigen-binding fragment thereof that specifically binds to TIM-3, comprising culturing the host cell of claim 10.

12. A kit for measuring a TIM-3 expression level comprising the monoclonal antibody or antigen-binding fragment thereof of claim 1.

13. A method for monitoring a TIM-3 expression level comprising:
i) contacting the monoclonal antibody or antigen-binding fragment thereof of claim 1 with a biological sample isolated from a subject; and
ii) measuring a TIM-3 expression level bound to the monoclonal antibody or antigen-binding fragment thereof in the biological sample through antigen-antibody complex formation.

14. The method of claim 13, wherein the biological sample is at least one selected from the group consisting of whole blood, serum, plasma, saliva, urine, tissue and cell.
